(19) Europäisches Patentamt

European Patent Office

Office européen des brevets

(11) **EP 0 759 293 B1**

(12) **FASCICULE DE BREVET EUROPEEN**

(45) Date de publication et mention
de la délivrance du brevet:
**07.10.1998 Bulletin 1998/41**

(51) Int. Cl.⁶: **A61K 7/48**, A61K 7/06

(21) Numéro de dépôt: 96401451.8

(22) Date de dépôt: **01.07.1996**

(54) **Composition stable contenant une enzyme**

Stabile Zusammensetzung enthaltend ein Enzym

Stable composition containing an enzyme

(84) Etats contractants désignés:
**DE ES FR GB IT**

(30) Priorité: **25.07.1995 FR 9509027**

(43) Date de publication de la demande:
**26.02.1997 Bulletin 1997/09**

(73) Titulaire: **L'OREAL**
**75008 Paris (FR)**

(72) Inventeurs:
• **Afriat, Isabelle**
**75014 Paris (FR)**
• **Gagnebien, Didier**
**92320 Chatillon (FR)**

(74) Mandataire: **Lhoste, Catherine**
**L'OREAL,**
**D.P.I.,**
**90 rue du Général Roguet**
**92583 Clichy Cédex (FR)**

(56) Documents cités:
EP-A- 0 550 279        EP-A- 0 586 303
EP-A- 0 623 342        EP-A- 0 755 672
EP-A- 0 755 673        FR-A- 2 287 899
LU-A-   61 123          NN-A-      672
NN-A-   55 672          US-A- 5 133 968

• PATENT ABSTRACTS OF JAPAN vol. 14, no. 57
(C-0684) & JP-A-01 283213 (TADAO SHIRAISHI),
14 Novembre 1989,
• PATENT ABSTRACTS OF JAPAN vol. 14, no. 453
(C-0764) & JP-A-02 178210 (KANEBO LTD), 11
Juillet 1990,
• CHEMICAL ABSTRACTS, vol. 87, no. 9, 29 Août
1977 Columbus, Ohio, US; abstract no. 66843,
XP002001820 & RIV. ITAL.
ESSENZE,PROFUMI,PIANTE
OFF.,AROMI,SAPONI,COSMET.,AEROSOL, vol.
59, no. 5, 1977, pages 208-211, TRANOS, D:
"behavior of enzymes ...water activity"

**Description**

La présente invention a pour objet une composition à application topique contenant une enzyme, destinée à être utilisée en particulier pour nettoyer et/ou soigner et/ou protéger la peau et/ou les fibres kératiniques.

Il est connu d'introduire dans les compositions cosmétiques et/ou dermatologiques des enzymes, et notamment des protéases utilisées pour leurs propriétés protéolytiques. Ces enzymes sont recherchées dans le domaine cosmétique pour leur pouvoir lissant et nettoyant, et leur aptitude à éliminer les cellules mortes de la peau.

Malheureusement, les enzymes présentent l'inconvénient d'être instables en milieu aqueux et d'être facilement dégradées ou modifiées sous l'influence de l'eau. Elles perdent ainsi rapidement de leur activité au cours du temps et cette instabilité va à l'encontre de l'efficacité recherchée.

Aussi, différents moyens ont été envisagés pour pallier à cet inconvénient. En particulier, il a été envisagé de mettre une enzyme dans une composition pulvérulente (voir le document JP-A-63-130514). D'ailleurs, la plupart des produits de nettoyage de la peau contenant une enzyme se présentent sous cette forme. Il a été aussi envisagé d'utiliser les enzymes, sous forme immobilisée sur des supports polymériques (voir le document JP-A-61-207499) ou dans des microcapsules (voir le document JP-A-61-254244). Malheureusement, certains de ces moyens nécessitent une mise en oeuvre particulière, ce qui accroît le coût et le temps de préparation de la composition.

Une autre solution consiste à les incorporer dans un milieu liquide anhydre (voir le document US-A-5322683). Malheureusement, cette solution limite la forme galénique de la composition et ne permet pas l'incorporation d'actifs hydrophiles.

Il subsiste donc le besoin d'une composition pour application topique contenant des enzymes, dans laquelle ces dernières conservent toutes leurs propriétés et donc leur efficacité au cours du temps.

La demanderesse a maintenant trouvé de manière inattendue que l'utilisation d'au moins un polyol liant l'eau dans une composition topique contenant une enzyme, en quantité efficace pour obtenir une valeur d'activité en eau de la composition, inférieure ou égale à 0,85, et d'au moins un agent structurant permet d'éviter la dégradation de l'enzyme.

Aussi, la présente invention a pour objet une composition stable à application topique contenant au moins une enzyme et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce qu'elle comprend au moins un polyacrylate de glycéryle complexé avec plus de 65 % de glycérine et/ou de propylène glycol et moins de 35 % en poids d'eau et/ou une huile.

L'invention se rapporte aussi à une composition stable à application topique sous forme d'une solution ou d'une émulsion, contenant au moins une enzyme et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce qu'elle comprend au moins un polyacrylate de glycéryle complexé avec plus de 65 % de glycérine et/ou de propylène glycol et moins de 35 % en poids d'eau et/ou une huile.

Certes, il est connu que la teneur en eau peut avoir une influence sur la stabilité des enzymes, mais il n'a jamais été décrit ni suggéré que la seule présence de polyol et d'un agent structurant puisse éviter leur dégradation. Ainsi, le document de D. Tzanos (Behavior of enzymes by controlling the medium water activity ; Riv. Ital. Essenze, Profumi, Piante Off., Aromi, Saponi, Cosmet., Aerosol, 1977, vol.59, n°5, pages 208-211) incite l'homme du métier à utiliser des tensioactifs pour la stabilisation des enzymes en milieu aqueux ou à fixer les enzymes sur un support poreux. En revanche, il écarte l'homme du métier d'utiliser des glycols.

Par ailleurs, le document US-A-5356800 décrit un procédé de stabilisation des enzymes consistant à utiliser un mélange comprenant un alcool ou un glycol, une alkyldiamine oxyéthylénée et un oxyde d'amine. Selon ce document, la stabilisation des enzymes ne peut être obtenue qu'en utilisant le mélange revendiqué.

En outre, le document JP-A-01-283213 décrit une composition de nettoyage contenant une enzyme et un polyol. Selon ce document, l'activité enzymatique est stabilisée par addition d'une protéine telle que le collagène, l'élastine ou l'albumine.

Le document FR-A-1397399 décrit un procédé pour stabiliser les protéases consistant à utiliser un mélange de polyol et de sel de calcium. Selon ce document, la présence de sel de calcium est indispensable à la stabilisation de la protéase.

Par ailleurs, il est connu du document J. Soc. Cosm. Chem. Jap., 1993, 27(3), p.276-288 que l'on peut stabiliser les protéases en les modifiant chimiquement et que l'addition de polyols contribue à améliorer la stabilité de la protéase modifiée. Selon ce document, la modification chimique est nécessaire pour obtenir la stabilisation des enzymes.

Or, il a été maintenant trouvé que, dans le cas des compositions topiques, les polyols utilisés en une quantité suffisante et en association avec un agent structurant peuvent empêcher la dégradation des actifs sensibles à l'eau.

La présente invention a encore pour objet l'utilisation dans une composition à application topique exempte de sel de calcium et contenant au moins une enzyme, d'au moins un polyol en une quantité efficace pour obtenir une valeur d'activité en eau de la composition, inférieure ou égale à 0,85, et d'au moins un polyacrylate de glycéryle complexé avec plus de 65 % de glycérine et/ou de propylène glycol et moins de 35 % en poids d'eau et/ou une huile, en vue de stabiliser l'enzyme.

De préférence, le polymère est choisi parmi les polymères acryliques et méthacryliques.

De préférence, la quantité du ou des polyols doit être telle que la valeur d'activité en eau de la composition est inférieure ou égale à 0,7.

L'activité en eau $a_w$ d'un milieu contenant de l'eau est le rapport de la pression de vapeur d'eau du produit 《 $P_{H2O}$ produit 》 et de la pression de vapeur de l'eau pure 《 $P_{H2O}$ pur 》 à la même température. Elle peut être exprimée aussi comme le rapport du nombre de molécules d'eau 《 $N_{H2O}$ 》 sur le nombre de molécules totales 《 $N_{H2O} + N_{corps\ dissous}$ 》, qui tient compte de celles des corps dissous 《 $N_{corps\ dissous}$ 》.

Elle est donnée par les formules suivantes :

$$a_w = \frac{P_{H2O}\ produit}{P_{H2O}\ pur} = \frac{N_{H2O}}{N_{H2O} + N_{corps\ dissous}}$$

On peut utiliser différentes méthodes pour mesurer l'activité en eau. La plus courante est la méthode manométrique par laquelle on mesure directement la pression de vapeur.

De manière classique, une composition cosmétique ou dermatologique a une activité en eau située autour de 0,95 à 0,99. Une activité en eau inférieure à 0,85 représente une diminution notable de l'activité en eau.

Le polyol utilisé selon l'invention peut être notamment choisi parmi la glycérine et les glycols, en particulier le propylène glycol et les polyéthylène glycols.

La quantité de polyol(s) à utiliser dans la composition de l'invention dépend du type de composition (gel ou émulsion) et des autres constituants présents dans la composition. Cette quantité doit être suffisante pour atteindre la valeur recherchée d'activité en eau. Le ou les polyols utilisés selon l'invention sont de préférence présents en une quantité d'au moins 30 % en poids, de préférence allant de 30 à 99,99 % en poids, et mieux de 60 à 80 % en poids par rapport au poids total de la composition.

Selon un mode de réalisation préféré de l'invention, le ou les polyols se trouvent totalement ou partiellement sous forme complexée avec un polymère acrylique ou méthacrylique. Le polymère peut également comprendre de l'eau liée, c'est-à-dire être complexé avec un mélange d'eau et de polyol(s).

On entend par polymère acrylique ou méthacrylique un homopolymère ou un copolymère d'acide acrylique ou méthacrylique ou un homopolymère ou un copolymère d'un dérivé d'acide acrylique ou méthacrylique.

La quantité de polymères avec le ou les polyols et éventuellement l'eau liée, dans la composition selon l'invention va de préférence de 70 à 99,99 % en poids, et mieux de 80 à 95 % en poids par rapport au poids total de la composition.

On peut citer comme homopolymère complexant l'eau et les polyols, ceux vendus sous les dénominations de Norgel et de Lubrajel CG par la société Guardian. Ces polymères sont des polyacrylates de glycéryle complexés avec plus de 65 % de glycérine et/ou de propylène glycol et moins de 35 % en poids d'eau. Ces polymères apportent le polyol et l'eau complexés, et éventuellement jouent en outre le rôle de gélifiant de la composition.

Les tests comparatifs présentés ci-dessous montrent que seules les compositions ayant une valeur d'activité en eau liée au plus égale à 0,85 permettent une bonne conservation de l'activité enzymatique des enzymes.

Les enzymes utilisées selon l'invention sont notamment les lactoperoxydases, les lipases, les protéases, les phospholipases et les cellulases.

Le ou les enzymes utilisées selon l'invention sont de manière avantageuse une protéase. Elles peuvent être choisies par exemple parmi celle vendue sous la dénomination commerciale "Subtilisine SP 544" par la société Novo Nordisk et celle vendue sous la dénomination commerciale "Lysoveg" par la société Laboratoires Sérobiologiques de Nancy.

Dans la composition selon l'invention, l'enzyme peut être utilisée de manière avantageuse en une quantité allant de 0,001 à 15 % en poids, de préférence de 0,01 à 10 % en poids, et mieux de 0,05 à 5 % en poids par rapport au poids total de la composition.

Comme huiles utilisables dans l'invention, on peut citer les huiles minérales (huile de vaseline), les huiles végétales (huile de jojoba), les huiles animales, les huiles de synthèse (oléate de décyle), les huiles siliconées (cyclométhicone, polydiméthylsiloxane, diméthicone) et les huiles fluorées (perfluoropolyéthers). La ou les huiles peuvent être présentes en une quantité allant de 5 à 60 %, et de préférence de 5 à 40 % en poids par rapport au poids total de la composition.

En outre, la composition selon l'invention peut contenir un ou plusieurs sels dont la présence va encore améliorer la stabilité de l'actif qu'elle contient. Comme sels, on peut citer en particulier les sels de magnésium et les sels de sodium, et plus spécialement le sulfate de magnésium, le chlorure de magnésium et le chlorure de sodium. Le ou les sels peuvent être présents en une quantité allant de 0,1 à 30 % et de préférence de 2 à 12 % en poids par rapport au poids total de la composition.

La composition selon l'invention contient un milieu topiquement acceptable, c'est-à-dire compatible avec la peau et les cheveux, et constitue notamment des compositions de nettoyage, de protection, de traitement ou de soin de la peau et/ou des cheveux, en particulier pour le visage, pour le cou, pour les mains, pour les cheveux, pour le cuir chevelu ou pour le corps, ainsi que pour les cils.

Aussi, l'invention a encore pour objet l'utilisation cosmétique de la composition selon l'invention pour nettoyer et/ou protéger la peau et/ou les fibres kératiniques, c'est-à-dire les cheveux et/ou les cils.

La présente invention a aussi pour objet une composition nettoyante pour la peau et/ou les fibres kératiniques contenant au moins une enzyme et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce qu'elle comprend au moins un polyacrylate de glycéryle complexé avec plus de 65 % de glycérine et/ou de propylène glycol et moins de 35 % en poids d'eau et/ou une huile.

L'invention a enfin pour objet un procédé cosmétique et/ou dermatologique pour nettoyer et/ou protéger la peau et/ou les fibres kératiniques, caractérisé en ce qu'il consiste à appliquer sur la peau et/ou les fibres kératiniques une composition exempte de sel de calcium, contenant au moins une enzyme, au moins un polyol présent en une quantité efficace pour obtenir une valeur d'activité en eau de la composition, inférieure ou égale à 0,85, et au moins un polyacrylate de glycéryle complexé avec plus de 65 % de glycérine et/ou de propylène glycol et moins de 35 % en poids d'eau et/ou une huile en vue de stabiliser l'enzyme.

La composition selon l'invention peut se présenter notamment sous forme d'une solution, d'un gel, d'une émulsion eau-dans-huile ou huile-dans-eau constituant des crèmes, des onguents, des lotions ou des laits. Cette composition peut comprendre aussi des microcapsules, des microparticules, ou des dispersions vésiculaires de type ionique et/ou non ionique. Ces différentes formes de composition sont préparées selon les méthodes usuelles.

Ces compositions constituent notamment des crèmes de protection, de traitement ou de soin pour le visage, pour les mains, pour les pieds, des laits corporels de protection ou de soin, des lotions, gels ou mousses pour le soin de la peau, des cheveux, des muqueuses et du cuir chevelu.

Lorsque la composition de l'invention est une émulsion, la proportion de la phase grasse peut aller de 10 % à 80 % en poids, et de préférence de 20 % à 40 % en poids par rapport au poids total de la composition. L'émulsion comprend de préférence au moins un agent dispersant choisi parmi les émulsionnants, les vésicules et les particules. Les huiles, les émulsionnants et éventuellement les coémulsionnants utilisés dans la composition sous forme d'émulsion sont choisis parmi ceux classiquement utilisés dans les domaines cosmétique et dermatologique. L'émulsionnant et le coémulsionnant sont par exemple présents, dans la composition, en une proportion allant de 1 % à 10 % en poids, et de préférence de 2 à 6 % en poids par rapport au poids total de la composition.

De façon connue, la composition de l'invention peut contenir également des adjuvants habituels dans les domaines cosmétique et dermatologique, tels que les tensioactifs, notamment les tensioactifs moussants, les actifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 % à 10 % du poids total de la composition. Ces adjuvants, selon leur nature, peuvent être introduits dans la phase grasse, dans la phase aqueuse et/ou dans les vésicules lipidiques.

La phase grasse peur comprendre en plus des huiles indiquées ci-dessus des matières grasses telles que des alcools gras, des acides gras (acide stéarique), des cires (cire de silicone).

Comme tensioactifs moussants utilisables dans l'invention, on peut citer le cocoamphodiacétate de disodium (Miranol C2M vendu par la société Rhône-Poulenc) et le décyl éther de glucose à 55 % dans l'eau (Oramix NS10 vendu par la société Seppic). La teneur en eau de ces matières premières fait partie de la quantité totale d'eau dans la composition.

Comme émulsionnants utilisables dans l'invention, on peut citer par exemple les émulsionnants siliconés comme les alkyldiméthicone copolyols tels que le cétyldiméthicone copolyol vendu par la société Goldschmidt sous la dénomination Abil EM-90, ou le mélange de diméthicone copolyol et cyclométhicone, vendu par la société Dow Corning sous la dénomination 3225C Formulation Aid.

Comme actifs hydrophiles, on peut utiliser les protéines ou les hydrolysats de protéine, les acides aminés, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, le rétinol (vitamine A) et ses dérivés, les acides gras essentiels, les céramides, les huiles essentielles.

**Test de stabilité de l'activité enzymatique :**

On a déterminé l'activité enzymatique d'une enzyme contenue dans un gel aqueux selon l'invention et dans deux gels comparatifs en utilisant la méthode à la caséine. Selon cette méthode, la caséine utilisée comme substrat est hydrolysée par l'enzyme en libérant des acides aminés qui sont ensuite quantifiés par colorimétrie à l'aide du réactif de Folin-Ciocalteu. L'absorbance colorimétrique lue est d'autant plus grande que la quantité d'enzyme est importante.

Les gels testés contenaient 1 % p/p de protéase (protéase SP 544) et avaient la composition suivante :

- Gel I (selon l'invention) : 99 % de Norgel (soit 0,99 % de polymère acrylique, 66,3 % de polyol et 30,7 % d'eau) et 1 % de protéase.
- Gel II (comparatif) : 99 % d'alginate de propylène glycol estérifié à 80-85 %, à 0,5 % dans l'eau et 1 % de protéase.
- Gel III (comparatif) : 99 % de polysaccharide (Fucogel 1000 : Biosaccharide gum-1 vendu par la société Solabia à base de fucose, galactose et d'acide galacturonique) et 1 % de protéase.

Le tableau suivant donne les résultats en pourcentage d'activité enzymatique restante après deux mois :

| Gel | Activité en eau du gel $a_w$ | % d'activité enzymatique |
|---|---|---|
| Gel I | 0,65 | 71 % |
| Gel II | 0,989 | 0 % |
| Gel III | 0,967 | 0 % |

Ces résultats montrent que seul le gel I selon l'invention permet la conservation de l'activité enzymatique de la protéase.

Les exemples ci-après de compositions selon l'invention, sont donnés à titre d'illustration et sans caractère limitatif. Les quantités y sont données en % en poids.

**Exemple 1: Gel**

| | |
|---|---|
| Norgel | 85 % |
| Subtilisine SP 544 | 0,1 % |
| Eau | qsp 100 % |

On obtient un gel translucide, utilisable comme gel exfoliant. Son activité en eau est de 0,735 ± 0,05.

Après 2 mois à la température ambiante, l'activité enzymatique de la subtilisine SP 544 est encore de 80 %.

**Exemple 2 : Emulsion eau-dans-huile**

| Phase aqueuse : | |
|---|---|
| Norgel | 71,5 % |
| NaCl | 0,5 % |

| Phase huileuse : | |
|---|---|
| Cétyldiméthicone copolyol (Abil EM-90 vendu par la société Goldschmidt)(émulsionnant) | 2 % |
| Huile de jojoba | 4 % |
| Huile de vaseline | 10 % |
| Polydiméthylsiloxane | 8 % |
| Oléate de décyle | 3,9 % |

(suite)

| Phase huileuse : | |
|---|---|
| Subtilisine SP544 | 0,1 % |

Le mode opératoire pour préparer l'émulsion est le suivant: On prépare la phase aqueuse d'une part et la phase huileuse d'autre part, et on émulsionne la phase aqueuse dans la phase huileuse à température ambiante sous agitation à l'homogénéisateur.

On obtient une crème blanche apte à faciliter l'élimination des cellules de la peau et à éclaircir le teint. Son activité en eau est de 0,62 ± 0,02.

Après 2 mois à la température ambiante, l'activité enzymatique de la subtilisine SP 544 est encore de 100 %.

**Exemple 3 : Gel de nettoyage**

| Subtilisine SP544 | 0,04 % |
|---|---|
| Norgel | 83 % |
| Miranol C2M (vendu par la société Rhône-Poulenc) | 16 % |
| Eau | qsp 100 % |

On obtient un gel de nettoyage moussant pour le visage et le corps, rinçable à l'eau. Son activité en eau est de 0,67 ± 0,02.

**Exemple 4 : Gel de nettoyage**

| Norgel | 88,97 % |
|---|---|
| Lysoveg | 0,03 % |
| Oramix NS10 (vendu par la société Seppic) | 11 % |

On obtient un gel de nettoyage moussant pour le visage et le corps, rinçable à l'eau. Son activité en eau est de 0,68 ± 0,02.

**Exemple 5 : Emulsion eau-dans-huile**

| Phase huileuse : | |
|---|---|
| Diméthicone copolyol et cyclométhicone (〈〈 3225C Formulation Aid 〉〉 vendu par Dow Corning) | 22,6 % |
| Diméthicone | 5 % |
| Huile minérale | 3 % |

| Phase aqueuse: | |
|---|---|
| Glycérine | 45,5 % |
| Subtilisine SP544 | 0,05 % |

(suite)

| Phase aqueuse: | |
| --- | --- |
| Sulfate de magnésium (stabilisant) | 2 % |
| Propylène glycol | 8 % |
| Eau | qsp 100 % |

L'émulsion est préparée de la même manière que dans l'exemple 2.

On obtient une crème blanche pour le lissage de la peau, dont l'activité en eau est de 0,63 ± 0,02.

Après 2 mois à la température ambiante, l'activité enzymatique de la subtilisine SP 544 est encore de 90 %.

**Exemple 6 : Emulsion eau-dans-huile**

| Phase huileuse : | |
| --- | --- |
| Diméthicone copolyol et cyclométhicone (《 3225C Formulation Aid 》 vendu par Dow Corning) | 22,8 % |
| Diméthicone | 5 % |
| Octyl palmitate | 6,7 % |
| Amidon de maïs | 8 % |
| Nylon-12 | 5 % |

| Phase aqueuse : | |
| --- | --- |
| Glycérine | 8 % |
| Propylène glycol | 8 % |
| Chlorure de magnésium | 6 % |
| Subtilisine SP544 | 0,1 % |
| Eau | qsp 100 % |

L'émulsion est préparée de la même manière que dans l'exemple 2.

On obtient une crème blanche pour le lissage de la peau, dont l'activité en eau est de 0,75 ± 0,02.

**Revendications**

1. Composition stable à application topique contenant au moins une enzyme et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce qu'elle comprend au moins un polyacrylate de glycéryle complexé avec plus de 65 % de glycérine et/ou de propylène glycol et moins de 35 % en poids d'eau et/ou une huile.

2. Composition stable à application topique sous forme d'une solution ou d'une émulsion, contenant au moins une enzyme et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce qu'elle comprend au moins un polyacrylate de glycéryle complexé avec plus de 65 % de glycérine et/ou de propylène glycol et moins de 35 % en poids d'eau et/ou une huile.

3. Composition selon la revendication 1 ou 2, caractérisée en ce que le polyol est présent en une quantité efficace pour obtenir une valeur d'activité en eau de la composition, inférieure ou égale à 0,7.

7

4. Composition selon l'une quelconque des revendications précédentes, caractérisée en ce que le polyol est présent en une quantité d'au moins 30 % en poids par rapport au poids total de la composition.

5. Composition selon l'une quelconque des revendications précédentes, caractérisée en ce que le polyol est choisi dans le groupe comprenant la glycérine et les glycols.

6. Composition selon l'une quelconque des revendications précédentes, caractérisée en ce que le polymère, le polyol et l'eau liée sont présents en une quantité allant de 70 à 99,99 % en poids par rapport au poids total de la composition.

7. Composition selon l'une quelconque des revendications précédentes, caractérisée en ce que l'enzyme est une protéase.

8. Composition selon l'une quelconque des revendications précédentes, caractérisée en ce que l'enzyme est présente en une concentration allant de 0,001 à 15 % en poids par rapport au poids total de la composition.

9. Composition selon l'une quelconque des revendications précédentes, caractérisée en ce que l'huile est présente en une quantité allant de 5 à 60 % en poids par rapport au poids total de la composition.

10. Composition selon l'une quelconque des revendications précédentes, caractérisée en ce que l'huile est choisie parmi les huiles minérales, les huiles végétales, les huiles animales, les huiles de synthèse, les huiles siliconées et les huiles fluorées.

11. Composition selon l'une quelconque des revendications précédentes, caractérisée en ce qu'elle comprend en outre un sel de magnésium ou un sel de sodium.

12. Composition selon l'une quelconque des revendications précédentes, caractérisée en ce qu'elle comprend en outre au moins un adjuvant lipophile ou hydrophile choisi parmi les conservateurs, les antioxydants, les parfums, les charges, les filtres, les séquestrants, les huiles essentielles, les matières colorantes, les actifs hydrophiles ou lipophiles, et les vésicules lipidiques.

13. Composition selon l'une quelconque des revendications précédentes, caractérisée en ce qu'elle se présente sous forme d'émulsion et en ce qu'elle comprend, en outre, au moins un agent dispersant choisi parmi les émulsionnants, les vésicules et les particules.

14. Composition nettoyante pour la peau et/ou les fibres kératiniques contenant au moins une enzyme et au moins un polyol, caractérisée en ce qu'elle est exempte de sel de calcium, en ce que le polyol est présent en une quantité efficace pour obtenir une valeur d'activité en eau de la composition, inférieure ou égale à 0,85, et en ce quelle comprend au moins un polyacrylate de glycéryle complexé avec plus de 65 % de glycérine et/ou de propylène glycol et moins de 35 % en poids d'eau et/ou une huile.

15. Utilisation cosmétique de la composition selon les revendications 1 à 13, pour nettoyer et/ou protéger la peau et/ou les fibres kératiniques.

16. Utilisation dans une composition à application topique exempte de sel de calcium et contenant au moins une enzyme, d'au moins un polyol en une quantité efficace pour obtenir une valeur d'activité en eau de la composition, inférieure ou égale à 0,85, et d'au moins un polyacrylate de glycéryle complexé avec plus de 65 % de glycérine et/ou de propylène glycol et moins de 35 % en poids d'eau et/ou une huile, en vue de stabiliser l'enzyme.

17. Procédé cosmétique pour nettoyer et/ou protéger la peau et/ou les fibres kératiniques, caractérisé en ce qu'il consiste à appliquer sur la peau et/ou les fibres kératiniques une composition exempte de sel de calcium, contenant au moins une enzyme, au moins un polyol présent en une quantité efficace pour obtenir une valeur d'activité en eau de la composition, inférieure ou égale à 0,85, et au moins un polyacrylate de glycéryle complexé avec plus de 65 % de glycérine et/ou de propylène glycol et moins de 35 % en poids d'eau et/ou une huile, en vue de stabiliser l'enzyme.

**Claims**

1. Stable composition for topical application containing at least one enzyme and at least one polyol, characterized in that the said composition contains no calcium salt, in that the polyol is present in a quantity which is effective for obtaining a water activity value of the composition of less than or equal to 0.85, and in that the said composition comprises at least one poly(glyceryl acrylate) complexed with more than 65% of glycerol and/or of propylene glycol and less than 35% by weight of water and/or one oil.

2. Stable composition for topical application in the form of a solution or of an emulsion containing at least one enzyme and at least one polyol, characterized in that the said composition contains no calcium salt, in that the polyol is present in a quantity which is effective for obtaining a water activity value of the composition of less than or equal to 0.85, and in that the said composition comprises at least one poly(glyceryl acrylate) complexed with more than 65% of glycerol and/or of propylene glycol and less than 35% by weight of water and/or one oil.

3. Composition according to Claim 1 or 2, characterized in that the polyol is present in a quantity which is effective for obtaining a water activity value of the composition of less than or equal to 0.7.

4. Composition according to any one of the preceding claims, characterized in that the polyol is present in a quantity of at least 30% by weight relative to the total weight of the composition.

5. Composition according to any one of the preceding claims, characterized in that the polyol is chosen from the group consisting of glycerol and glycols.

6. Composition according to any one of the preceding claims, characterized in that the polymer, the polyol and the bound water are present in a quantity ranging from 70 to 99.99% by weight, relative to the total weight of the composition.

7. Composition according to any one of the preceding claims, characterized in that the enzyme is a protease.

8. Composition according to any one of the preceding claims, characterized in that the enzyme is present in a concentration ranging from 0.001 to 15% by weight relative to the total weight of the composition.

9. Composition according to any one of the preceding claims, characterized in that the oil is present in a quantity ranging from 5 to 60% by weight, relative to the total weight of the composition.

10. Composition according to any one of the preceding claims, characterized in that the oil is chosen from mineral oils, vegetable oils, animal oils, synthetic oils, silicone oils and fluorinated oils.

11. Composition according to any one of the preceding claims, characterized in that it additionally comprises a magnesium salt or a sodium salt.

12. Composition according to any one of the preceding claims, characterized in that it additionally comprises at least one lipophilic or hydrophilic adjuvant chosen from preservatives, antioxidants, fragrances, fillers, screening agents, sequestering agents, essential oils, colouring substances, hydrophilic or lipophilic active agents and lipid vesicles.

13. Composition according to any one of the preceding claims, characterized in that it is provided in the form of an emulsion and in that it additionally comprises at least one dispersant chosen from emulsifiers, vesicles and particles.

14. Cleansing composition for the skin and/or keratinous fibres, containing at least one enzyme and at least one polyol, characterized in that the said composition contains no calcium salt, in that the polyol is present in a quantity which is effective for obtaining a water activity value of the composition of less than or equal to 0.85, and in that the said composition comprises at least one poly(glyceryl acrylate) complexed with more than 65% of glycerol and/or of propylene glycol and less than 35% by weight of water and/or one oil.

15. Cosmetic use of the composition according to Claims 1 to 13 for cleansing and/or protecting the skin and/or keratinous fibres.

16. Use, in a composition for topical application which contains no calcium salt and contains at least one enzyme, of at least one polyol in a quantity which is effective for obtaining a water activity value of the composition of less than or equal to 0.85, and of at least one poly(glyceryl acrylate) complexed with more than 65% of glycerol and/or of propylene glycol and less than 35% by weight of water and/or one oil, for the purpose of stabilizing the enzyme.

17. Cosmetic method of cleansing and/or protecting the skin and/or keratinous fibres, characterized in that it consists in applying to the skin and/or keratinous fibres a composition which contains no calcium salt and contains at least one enzyme, at leapt one polyol present in a quantity which is effective for obtaining a water activity value of the composition of less than or equal to 0.85, and at least one poly(glyceryl acrylate) complexed with more than 65% of glycerol and/or of propylene glycol and less than 35% by weight of water and/or one oil, for the purpose of stabilizing the enzyme.

**Patentansprüche**

1. Stabile Zusammensetzung für die topische Anwendung, die mindestens ein Enzym und mindestens einen mehrwertigen Alkohol enthält,
dadurch gekennzeichnet, daß
sie kein Calciumsalz enthält und daß der mehrwertige Alkohol in einer Menge enthalten ist, die für die Erzielung eines Aktivitätswerts in Wasser der Zusammensetzung von kleiner als oder gleich 0,85 wirksam ist, und daß sie mindestens ein Polyglycerinacrylat, das mit mehr als 65 Gew.-% Glycerin und/oder Propylenglykol und weniger als 35 Gew.-% Wasser komplexiert ist, und/oder mindestens ein Öl enthält.

2. Stabile Zusammensetzung für die topische Anwendung in Form einer Lösung oder einer Emulsion, die mindestens ein Enzym und mindestens einen mehrwertigen Alkohol enthält, dadurch gekennzeichnet, daß
sie kein Calciumsalz enthält und daß der mehrwertige Alkohol in einer Menge enthalten ist, die für die Erzielung eines Aktivitätswerts in Wasser der Zusammensetzung von kleiner als oder gleich 0,85 wirksam ist, und daß sie mindestens ein Polyglycerinacrylat, das mit mehr als 65 Gew.-% Glycerin und/oder Propylenglykol und weniger als 35 Gew.-% Wasser komplexiert ist, und/oder mindestens ein Öl enthält.

3. Zusammensetzung nach Anspruch 1 oder 2, dadurch gekennzeichnet, daß der mehrwertige Alkohol in einer Menge enthalten ist, die zur Erzielung eines Aktivitätswerts in Wasser der Zusammensetzung von kleiner als oder gleich 0,7 wirksam ist.

4. Zusammensetzung nach einem der vorhergehenden Ansprüche, dadurch gekennzeichnet, daß der mehrwertige Alkohol in einer Menge von mindestens 30 Gew.-%, bezogen auf das Gesamtgewicht der Zusammensetzung, enthalten ist.

5. Zusammensetzung nach einem der vorhergehenden Ansprüche, dadurch gekennzeichnet, daß der mehrwertige Alkohol unter Glycerin und Glykolen ausgewählt ist.

6. Zusammensetzung nach einem der vorhergehenden Ansprüche, dadurch gekennzeichnet, daß das Polymer, der mehrwertige Alkohol und das gebundene Wasser in einer Menge von 70 bis 99,99 Gew.-%, bezogen auf das Gesamtgewicht der Zusammensetzung, enthalten sind.

7. Zusammensetzung nach einem der vorhergehenden Ansprüche, dadurch gekennzeichnet, daß das Enzym eine Protease ist.

8. Zusammensetzung nach einem der vorhergehenden Ansprüche, dadurch gekennzeichnet, daß das Enzym in einer Konzentration von 0,001 bis 15 Gew.-%, bezogen auf das Gesamtgewicht der Zusammensetzung, enthalten ist.

9. Zusammensetzung nach einem der vorhergehenden Ansprüche, dadurch gekennzeichnet, daß das Öl in einer Menge von 5 bis 60 Gew.-%, bezogen auf das Gesamtgewicht der Zusammensetzung, enthalten ist.

10. Zusammensetzung nach einem der vorhergehenden Ansprüche, dadurch gekennzeichnet, daß das Öl unter anorganischen Ölen, pflanzlichen Ölen, tierischen Ölen, synthetischen Ölen, Siliconölen und fluorhaltigen Ölen ausgewählt ist.

11. Zusammensetzung nach einem der vorhergehenden Ansprüche, dadurch gekennzeichnet, daß sie außerdem ein Magnesiumsalz oder ein Natriumsalz enthält.

12. Zusammensetzung nach einem der vorhergehenden Ansprüche, dadurch gekennzeichnet, daß sie außerdem mindestens einen lipophilen oder hydrophilen Hilfsstoff enthält, der unter Konservierungsmitteln, Antioxidantien, Parfums, Füllstoffen, Filtern, Maskierungsmitteln, etherischen Ölen, Farbmitteln, hydrophilen oder lipophilen Wirkstoffen und Lipidvesikeln ausgewählt ist.

13. Zusammensetzung nach einem der vorhergehenden Ansprüche, dadurch gekennzeichnet, daß sie in Form einer Emulsion vorliegt und daß sie außerdem mindestens ein Dispergiermittel, das unter Emulgatoren, Vesikeln und Partikeln ausgewählt ist, enthält.

14. Reinigende Zusammensetzung für die Haut und/oder die Keratinfasern, die mindestens ein Enzym und mindestens einen mehrwertigen Alkohol enthält,
dadurch gekennzeichnet, daß
sie kein Calciumsalz enthält und daß der mehrwertige Alkohol in einer Menge enthalten ist, die für die Erzielung eines Aktivitätswerts in Wasser der Zusammensetzung von kleiner als oder gleich 0,85 wirksam ist, und daß sie mindestens ein Polyglycerinacrylat, das mit mehr als 65 Gew.-% Glycerin und/oder Propylenglykol und weniger als 35 Gew.-% Wasser komplexiert ist, und/oder mindestens ein Öl enthält.

15. Kosmetische Verwendung der Zusammensetzung nach den Ansprüchen 1 bis 13 für die Reinigung und/oder den Schutz der Haut und/oder der Keratinfasern.

16. Verwendung in einer Zusammensetzung zur topischen Anwendung, die kein Calciumsalz enthält und mindestens ein Enzym, mindestens einen mehrwertigen Alkohol in einer Menge, die zur Erzielung eines Aktivitätswerts in Wasser der Zusammensetzung von kleiner als oder gleich 0,85 wirksam ist, und mindestens ein Polyglycerinacrylat, das mit mehr als 65 Gew.-% Glycerin und/oder Propylenglykol und weniger als 35 Gew.-% Wasser komplexiert ist, und/oder mindestens ein Öl enthält, zur Stabilisierung des Enzyms.

17. Kosmetisches Verfahren für die Reinigung und/oder den Schutz der Haut und/oder der Keratinfasern,
dadurch gekennzeichnet, daß
auf die Haut und/oder die Keratinfasern eine Zusammensetzung aufgetragen wird, die kein Calciumsalz enthält und die mindestens ein Enzym, mindestens einen mehrwertigen Alkohol, der in einer Menge enthalten ist, die zur Erzielung eines Aktivitätswerts in Wasser der Zusammensetzung von kleiner als oder gleich 0,85 wirksam ist, und mindestens ein Polyglycerinacrylat, das mit mehr als 65 Gew.-% Glycerin und/oder Propylenglykol und weniger als 35 Gew.-% Wasser komplexiert ist, und/oder mindestens ein Öls enthält, zur Stabilisierung des Enzyms.